# EUROPEAN PATENT APPLICATION

(11) **EP 2 371 409 A1**
(43) Date of publication of application: **05.10.2011**
(21) Application number: 10394006.0
(22) Date of filing: 31.03.2010
(51) Int. Cl.: A61M 13/00, A61B 17/34, A61M 11/00, B05B 7/00, A61M 16/08

(54) **Insufflation of body cavities**

(71) Applicant: AeroSurgical Limited, Galway (IE)
(72) Inventor: Lillis, Claire Elizabeth, Galway, County Galway (IE); Duffy, Conor Paul, Roscahill, County Galway (IE); Power, Patrick Joseph, Moycullen, County Galway (IE)
(74) Representative: O'Brien, John Augustine

(57) **Abstract**

An apparatus for use in insufflation of a body cavity (5), such as through a trocar (6) is described. One such application is laparoscopic surgery. The device is also suitable for use in any situation involving insufflation of a body cavity such as in arthroscopies, pleural cavity insufflation (for example during thoracoscopy), retroperitoneal insufflations (for example retroperitoneoscopy), during hernia repair, during mediastinoscopy and any other such procedure involving insufflation. The apparatus comprises a reservoir I for storing an liquid solution, an aerosol generator (2) for aerosolising the solution, and a controller (3) for controlling operation of the aerosol generator (2). Aerosolised liquid solution (which main contain a pharmaceutical) is entrained with insufflation gas using a T-piece connector or housing (30) having an insufflation gas inlet (31) and an outlet (32). The connector (30) also comprises an aerosol supply conduit (34) for delivering the aerosol from the aerosol generator (2) into a mixing chamber (33) in which the aerosol is entrained with insufflation gas. The aerosol insufflation gas mixture passes out of the connector (30) through the outlet (32) and is delivered along the insufflation gas conduit (15) to the trocar (6) for delivery into a body cavity (5). The connector (30) causes a substantial sudden reduction in the velocity of the insufflation gas from the insufflation gas inlet as it enters the mixing chamber (33). The connector (30) also causes a gradual increase in the velocity of the insufflation gas with entrained aerosol between the mixing chamber (33) and the outlet (32).

## Description

### Background of the Invention

Laparoscopic surgery, also called minimally or less invasive surgery (MIS or LIS) or keyhole surgery is a modem surgical technique in which operations in the body are performed through small incisions as compared to the larger incisions needed in traditional surgical procedures. Gas such as carbon dioxide is delivered, via an insufflator, into a body cavity such as the abdomen leading to the formation of a pneumoperitoneum, thereby providing sufficient space for the surgeon to operate. The insufflator maintains the pneumoperitoneum and acts to renew the gas when leaks occur.

Gas such as carbon dioxide that is used for insufflation is both cold and dry and it is not surprising therefore those patients undergoing laparoscopic procedures often suffer a significant drop in core body temperature, which can result in considerable post-surgical pain and significant complications, such as cardiac stress, immunological and clotting problems, for the patient. By using standard thermo physical principles it has been shown that the major cause of patient heat loss is due to evaporation from the body acting to humidify the large volumes of dry insufflated gas at ATPD (Ambient Temperature Pressure Dry) passing into the body which is at BTPS (Body Temperature Pressure Saturated). If such heat loss could be minimised, post-operative pain and the significant side effects suffered by the patient could be considerably alleviated.

Various attempts have been made to condition insufflation gas by heating, humidifying and or filtering the gas. However in general, known insufflation gas conditioning systems suffer from one or more disadvantages including complexity of construction involving expensive monitoring devices, inaccurate control and/or difficulties in using them in a controlled working environment.

Some systems employ heat moisture exchangers (HME). These operate directly in the flow path of the insufflation gas and are therefore inherently susceptible to affecting pressure or flow, dependent upon their level of saturation and condition. Other systems require manual intervention to respond to patients needs by the adding of moisture. Other prior art devices require the cumbersome procedure of passing gas over and through non-heated or heated liquid containers. Such devices present the major drawback of impeding pressure measurement in the insufflation cavity.

Systems using conventional jet nebulisers or nebulisation catheters exhibit one or more of the following disadvantages: impaction of larger particles resulting in poor dispersion of aerosol and diminished dose, fogging in the body cavity thus reducing the surgeon's visibility, interference with insufflator settings increasing flow/pressure in the system.

This invention is directed towards providing an improved method and an apparatus for use in insufflation.

### Statements of Invention

According to the invention there is provided a connector for an insufflation system comprising a housing having a mixing chamber, an insufflator gas inlet to the mixing chamber, an aerosolised liquid inlet to the mixing chamber, an outlet for delivery of insufflation gas with aerosolised liquid entrained therein, the connector causing a substantial reduction in the velocity of insufflation gas from the insufflation gas inlet as it enters the mixing chamber and causing an increase in the velocity of the insufflation gas with entrained aerosol between the mixing chamber and the outlet.

In one embodiment the connector comprises a tapered portion leading from the mixing chamber to the outlet. The taper angle β of the tapered portion may be between about 10° and 30°.

In one embodiment the tapered portion has an entry with a first internal diameter (D₁) adjacent to the mixing chamber and a second internal diameter (D₂) at the outlet and the ratio of the first diameter to the second diameter is at least 2:1,at least 3:1,at least 4:1,at least 5:1.

The distance between the taper entry and the outlet defines a length (L) and the ratio of the length to the first diameter D₁ may be at least 2:1, at least 3:1, at least 4:1.

In one embodiment a central longitudinal axis L₁ at the insufflation gas inlet is coaxial with a central longitudinal axis L₀ at the outlet.

Alternatively a central longitudinal axis L₁ at the insufflation gas inlet may be above a central longitudinal axis L₀ at the outlet.

In one embodiment the connector comprises a mounting leg for mounting an aerosol generator device to the connector. There may be an interlock between the connector mounting leg and the aerosol generator device.

In one case the mounting leg is angled towards the inlet of the connector.

The mounting leg may be offset from a vertical axis by a tilt angle of at least 10°. Said tilt angle may be about 15°.

The invention also provides an apparatus from use in insufflation comprising a connector of the invention and an aerosol generator for aerosolising a liquid and entraining the aerosol within an insufflation gas.

In one case the aerosol generator comprises a vibratable member having a plurality of apertures extending between a first surface and a second surface. The first surface may be adapted to receive the fluid to be aerosolised. The aerosol generator may be configured to generate an aerosol at the second surface.

In one case the vibratable member is dome-shaped in geometry.

The vibratable member may comprise a stretched flat shape.

The vibratable member may comprise a piezoelectric element.

In one embodiment the apertures in the vibratable member are sized to aerosolise the first fluid by ejecting droplets of the first fluid such that the majority of the droplets by mass have a size of less than 5 micrometers.

The apertures in the vibratable member may be sized to aerosolise the first fluid by ejecting droplets of the first fluid such that the majority of the droplets by mass have a size of less than 3 micrometers.

The apertures in the vibratable member may be sized to aerosolise the first fluid by ejecting droplets of the first fluid such that the majority of the droplets by mass have a size in one range of less than 10 micrometers. A range band may be from I to 3 micrometers. A range band may be from 7 to 9 micrometers.

In one embodiment the apparatus comprises a controller to control the operation of the aerosol generator.

The controller may be configured to control the flow rate of the fluid to be aerosolised.

The controller may be configured to deliver different flow rates of aerosol at different stages of a surgical procedure.

The controller may be configured to deliver full flow at the start and/or end of a procedure.

The controller may be configured to deliver reduced flow during a procedure.

The controller may be set to deliver a pre-set amount of aerosol into insufflation gas.

The apparatus may comprise means for varying the pre-set amount of aerosol.

The means for varying the pre-set amount of aerosol may in one case comprise a user interface such as a keypad or switch.

The controller may be configured to control operation of the aerosol generator responsive to the insufflation gas.

In one case the controller is configured to control operation of the aerosol generator responsive to the flow rate of the insufflation gas.

In one embodiment the apparatus comprises a device to determine the fluid flow rate of the insufflation gas.

The determining device may comprise a flow sensor such as a flowmeter.

The device to determine the fluid flow rate may comprise a differential pressure sensor.

In one embodiment the apparatus comprises a humidity meter to measure the level of humidification of the insufflation gas.

There may be a feedback loop to the controller to control the output from the aerosol generator responsive to the level of humidification of the insufflation gas.

In another aspect the invention provides a method for carrying out a procedure involving insufflation comprising the steps of:
generating an insufflation gas;
reducing the velocity of the insufflation gas on entry to a mixing chamber;
aerosolising a fluid using an aerosol generator;
entraining the aerosol with the insufflation gas in the mixing chamber;
increasing the velocity of the insufflation gas with entrained aerosol; and
delivering the insufflation gas with entrained aerosol to a patient.

In one embodiment the method comprises the step of delivering the entrained fluid and insufflation gas into the body to insufflate at least part of the body.

The fluid may be a liquid solution.

The liquid solution may be saline having a salt concentration of greater than 1µM.

In one embodiment the fluid contains a therapeutic and/or prophylactic agent.

The agent may be one or more selected from the group comprising an analgestic, an anti-inflammatory, an anti-infective, an anaesthetic, an anticancer chemotherapy agent, and an anti-adhesion agent.

In one case the procedure is a laparascopic procedure.

### Brief Description of the Drawings

The invention will be more clearly understood from the following description of some embodiments thereof, given by way of example only, with reference to the accompanying drawings, in which:-
Fig. 1 is a perspective view of an apparatus according to the invention for use in a procedure involving insufflation of a body cavity, such as laparoscopic surgery;
Fig. 1A is a perspective view of another apparatus according to the invention;
Fig. 1B is a schematic illustration of part of an apparatus according to the invention;
Fig. 2 is a side elevational view of a T-piece connector and tubing assembly according to the invention;
Fig. 3 is an exploded view of the assembly of Fig. 2;
Fig. 4 is an enlarged cross sectional view of a T-piece connector according to the invention;
Fig. 5 is an enlarged view of a detail of Fig. 4;
Figs. 6 and 7 are views similar to Fig. 4;
Fig. 8 is an enlarged cross sectional view illustrating alternative T-piece connectors according to the invention;
Fig. 9 is a schematic illustration of a part of the apparatus of Fig. 1;
Fig. 10 is an exploded isometric view of an aerosol generator used in the invention;
Fig. 11 is a cross-sectional view of the assembled aerosol generator of Fig. 10;
Fig. 12 is a perspective view of a controller housing used in the apparatus of the invention;
Figs. 13(a) and 13(b) are graphs of DC voltage versus time and AC voltage versus time respectively to achieve a 100% aerosol output;
Figs. 14(a) and 14(b) are graphs of DC voltage versus time and AC voltage versus time respectively to achieve a 50% aerosol output - Fig 14(a) illustrates the waveform output from a microprocessor to a drive circuit and Fig 14(b) illustrates the waveform output from a drive circuit to a nebuliser;
Figs. 15(a) and 15(b) are graphs of DC voltage versus time and AC voltage versus time respectively to achieve a 25% aerosol output - Fig 15(a) illustrates the waveform output from a microprocessor to a drive circuit and Fig 15(b) illustrates the waveform output from a drive circuit to a nebuliser;
Fig 16 is a graph of AC voltage versus time; and illustrates an output waveform from a drive circuit to a nebuliser;
Fig. 17 is a graph of frequency versus current for another apparatus according to the invention;
Fig. 18 is a view similar to Fig. 1 of another apparatus of the invention;
Fig. 19 is a view similar to Fig. 1 of a further apparatus of the invention;
Fig. 20 is a view similar to Fig. 1 of a still further apparatus of the invention;
Fig. 21 is a view similar to Fig. 1 of another apparatus of the invention;
Fig. 22 is a view similar to Fig. 1 of a further apparatus of the invention;
Fig. 23 is a partially cross sectional view of a detail of the apparatus of Fig. 22;
Fig. 24 is a view similar to Fig. 1 of another apparatus of the invention;
Fig. 25 is a side elevational view of another system according to the invention;
Fig. 26 is a cross sectional view of another connector according to the invention; and

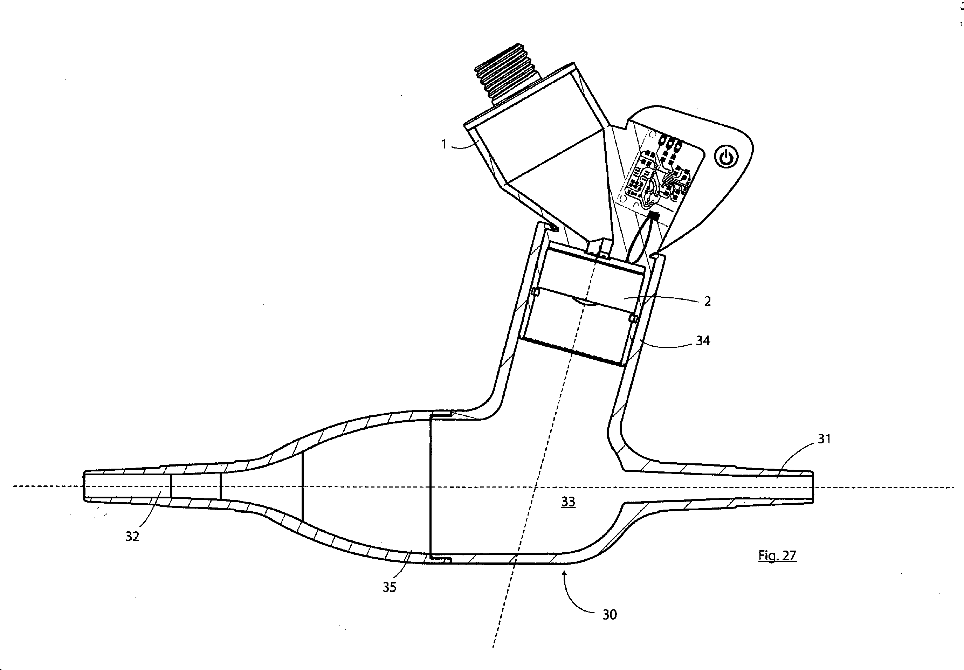

Fig. 27 is a cross sectional view of a further connector according to the invention.

### Detailed Description

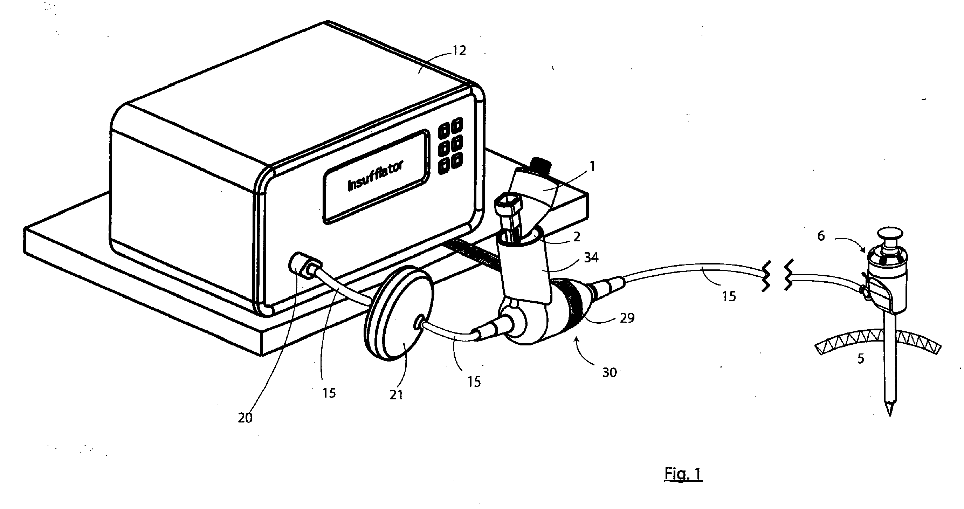

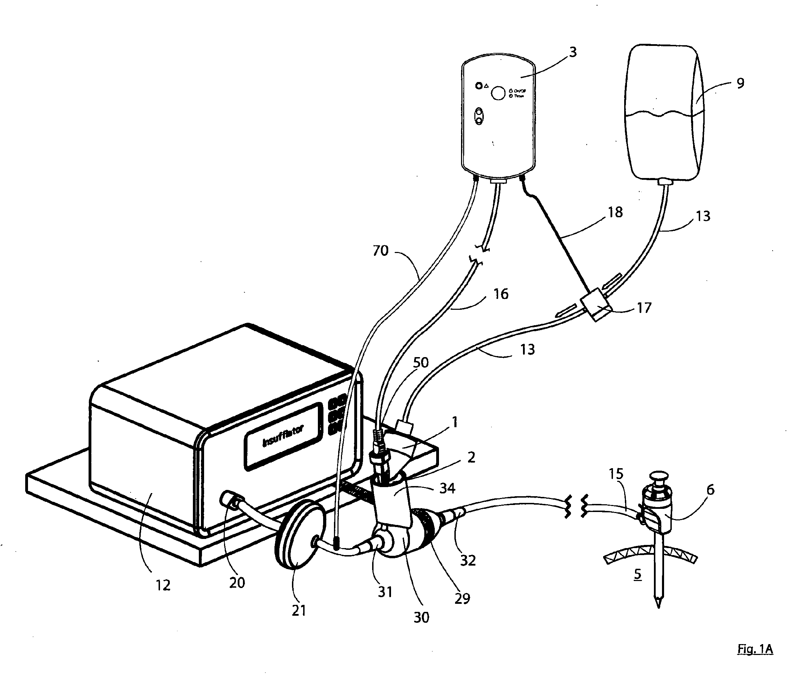

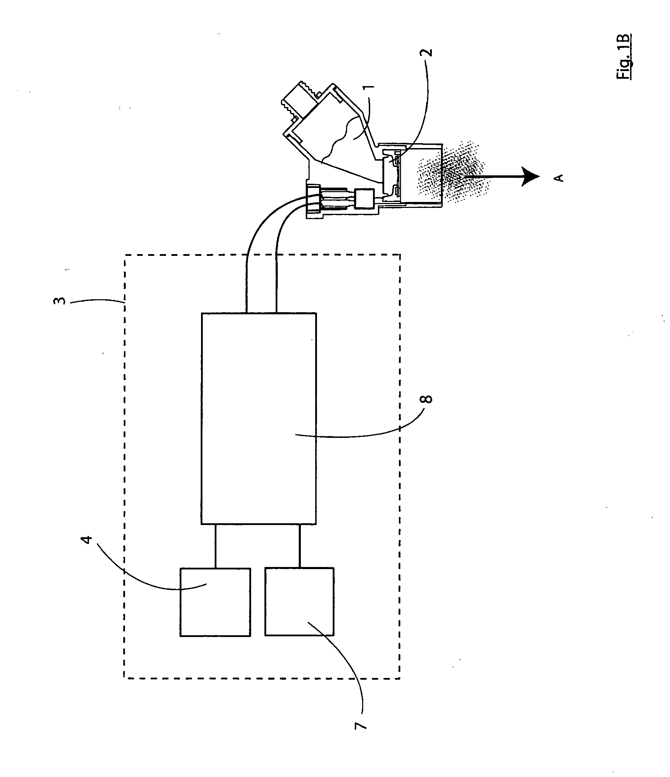

Referring to Fig. 1 there is illustrated an apparatus according to the invention for use in insufflation of a body cavity 5, in this case through a trocar 6. One such application is laparoscopic surgery. The device is also suitable for use in any situation involving insufflation of a body cavity such as in arthroscopies, pleural cavity insufflation (for example during thoracoscopy), retroperitoneal insufflations (for example retroperitoneoscopy), during hernia repair, during mediastinoscopy and any other such procedure involving insufflation.

The apparatus comprises a reservoir 1 for storing an liquid solution, an aerosol generator 2 for aerosolising the solution, and a controller 3 for controlling operation of the aerosol generator 2. In the invention aerosolised liquid solution is entrained with insufflation gas. The gas is any suitable insufflation gas such as carbon dioxide. Other examples of suitable insufflation gases are nitrogen, helium and xenon.

The insufflation gas is delivered into an insufflation gas tubing 15 by an insufflator 12. The insufflator 12 may be of any suitable type such as those available from Karl Storz, Olympus and Stryker. The insufflator 12 has an outlet 20 through which insufflation gas is delivered. A bacterial filter (sometimes called a transducer protector ― protecting the patient from insufflator contaminants and vice versa) 21 may be provided within the insufflator or, as illustrated, downstream of the insufflator outlet 20.

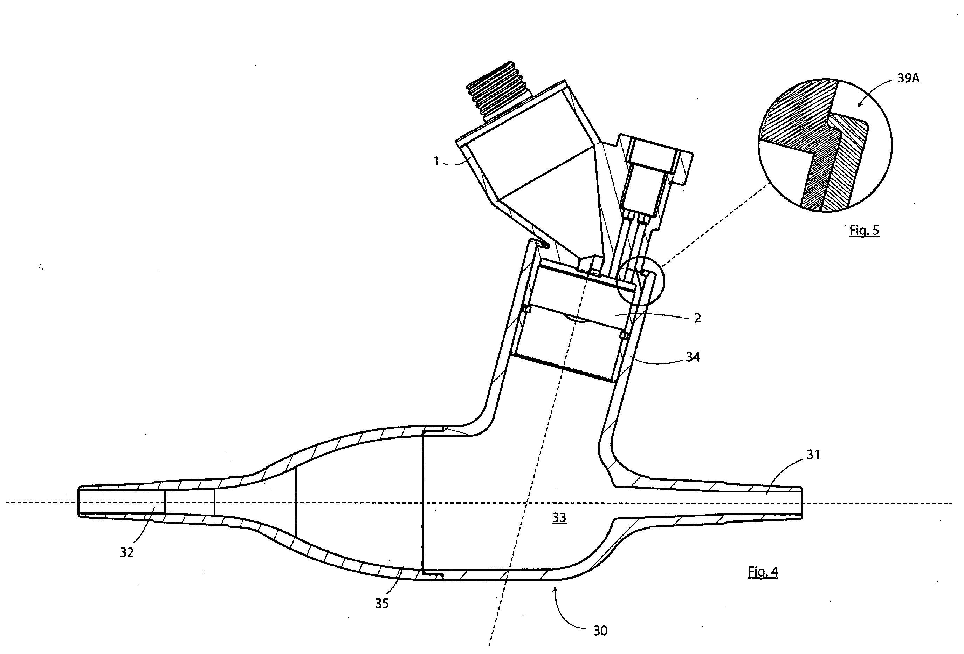

Referring in particular to Figs. 4 to 7 the invention provides a connector, in this case a T-piece connector or housing 30 having an insufflation gas inlet 31 and an outlet 32. The connector 30 also comprises an aerosol supply conduit 34 for delivering the aerosol from the aerosol generator 2 into a mixing chamber 33 in which the aerosol is entrained with insufflation gas. The aerosol insufflation gas mixture passes out of the connector 30 through the outlet 32 and is delivered along the insufflation gas conduit 15 to the trocar 6 for delivery into a body cavity 5.

The connector 30 causes a substantial sudden reduction in the velocity of the insufflation gas from the insufflation gas inlet as it enters the mixing chamber 33. The connector 30 also causes a gradual increase in the velocity of the insufflation gas with entrained aerosol between the mixing chamber 33 and the outlet 32.

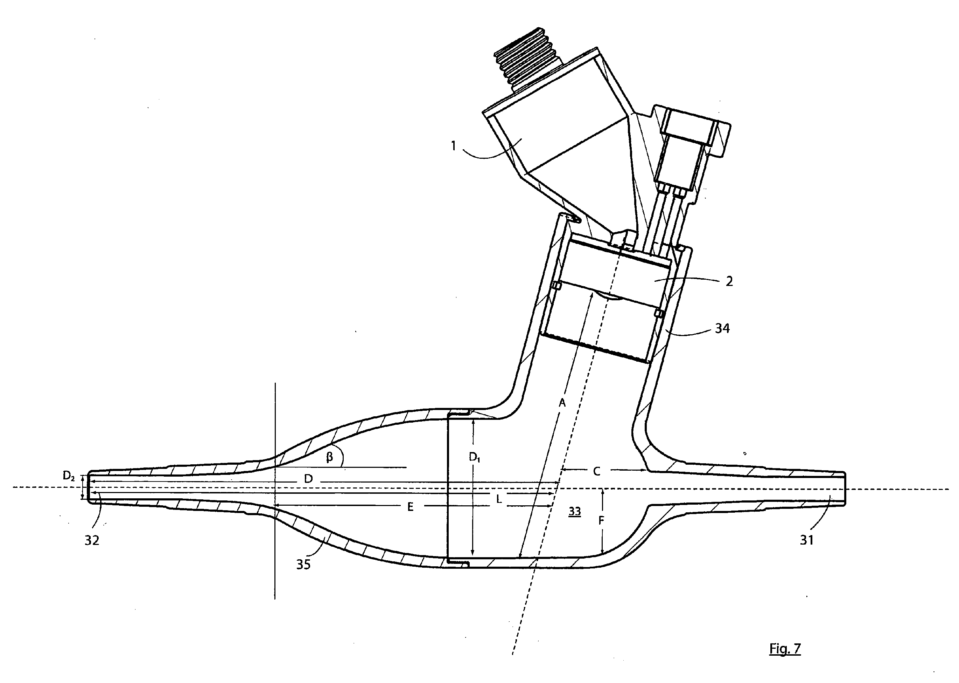

The connector 30 comprises a tapered portion 35 leading from the mixing chamber to the outlet. The taper angle β (Fig. 7) of the tapered portion 35 is between about 10° and 30°. The tapered portion 35 has an entry with a first diameter D₁ adjacent to the mixing chamber 33 and a second internal diameter D₂ at the outlet 32. The ratio of D₁:D₂ is preferably at least 2:1, at least 3:1, at least 4:1, at least 5:1. The distance between the taper entry and the outlet 32 defines a length L and the ratio of L:D₁ is at least 2:1; at least 3:1; at least 4:1.

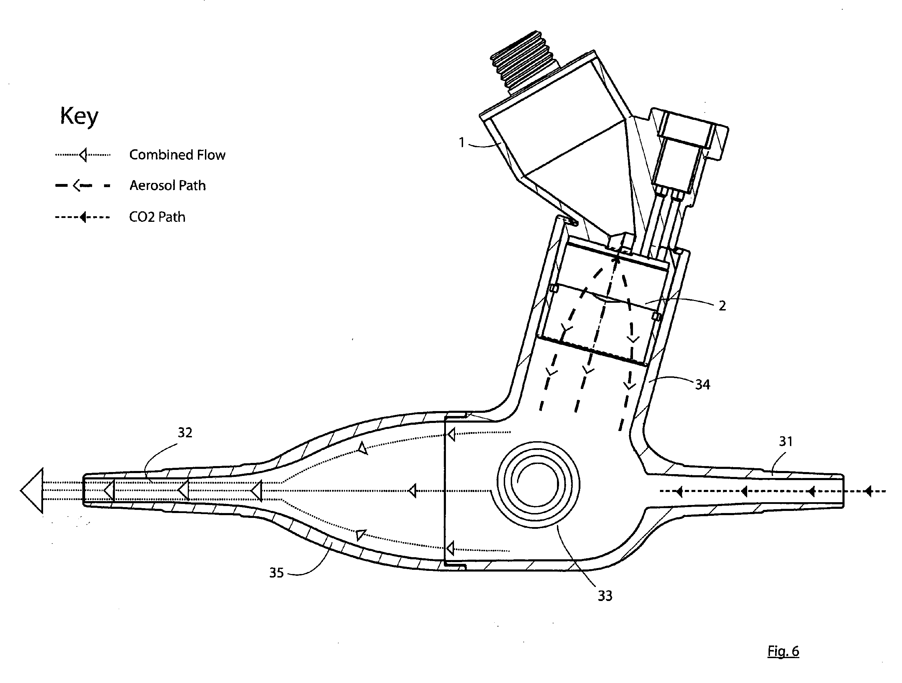

### Referring to Figs. 6 & 7:

Distance A: The height between the aerosol generator and bottom surface of the t-piece should be maximized (40mm-60mm) to avoid impaction of aerosol particles on bottom surface of t-piece. The lower velocity aerosol particle stream is re-directed as it crosses the axis of the higher velocity inlet gas flow so that the aerosol-inlet gas mixture flow direction is aligned with the device outlet. The lower wall of the device must be positioned to ensure sufficient space for the re-direction of the aerosol particle stream with minimal impaction on the inner surface.
Distance D₁: This inner diameter should be within 20-30mm range.
Distance C: This distance should be kept to a minimum, approx 10-20mm. Sudden expansion at end of narrow tubing leads to reduction of CO2 velocity reducing impact and collision of aerosol particles. Loss of aerosol through impaction on surfaces is proportional to the speed at which the impaction takes place. The sudden step change in diameter forces a rapid expansion of the gas flow and a proportional reduction is gas velocity through the aerosol mixing area for any given volume flow rate.
Distance D: From point of aerosol entry the t-piece should extend between 80-100mm to the output. The aerosol-gas mixture is susceptible to loss of aerosol particle content through impaction on the inner surfaces of the device. This loss is proportional to the speed at which the impaction takes place. The gas flow channel must be reduced to the diameter of the outlet which creates a proportional increase is gas velocity. The T-piece wall has a gentle taper to minimise aerosol collision.
Distance E: Point of aerosol entry to start of gentle taper should extend between 50-60mm.
Distance F: Height from lower wall of t-piece to centre axis of input port should be within 10-25mm range.

The aerosol supply conduit 34 defines a mounting leg for mounting the aerosol generator 2. The conduit 34 is tilted away from the outlet 32. The tilt angle is about 15° to induce the Coanda effect. The Coanda effect is the tendency of a fluid (in this case aerosol) to be attracted to a nearby surface.

The connector inlet and outlet legs may comprise steps so that the connector can be used with a range of insufflation gas tubing ― such as 6mm, 8mm and 10mm ID tubing.

Referring to Figs. 4 and 5 in particular the connector mounting leg 34 for the aerosol generator has an interlock 39A which locks the aerosol generator to the connector. This feature ensures that the - aerosol generator - cannot be removed and a different and incorrect one fitted. The interlock system shown is only one of a number of different techniques that could be employed to provide a permanent attachment. Such techniques include but are not limited to adhesive bonding, and welding such as spin welding.

The connector also defines a bracket support 29 to ensure that the weight of the device is supported and that the device remains horizontal which maximises the delivery to the patient of insufflation gas with entrained aerosolised liquid.

In Figs. 4 to 7 the axis of the inlet and outlets are co-linear. To reduce the possibility of impaction the axis of the inlet should not be below the axis of the outlet. The lower velocity aerosol particle stream is re-directed as it crosses the axis of the higher velocity inlet gas flow so that the aerosol-inlet gas mixture flow direction is aligned with the device outlet. The central axis at which the two streams meet should not be lower than the axis of the outlet to ensure sufficient space for the re-direction of the aerosol particle stream with minimal impaction on the lower inner surface.

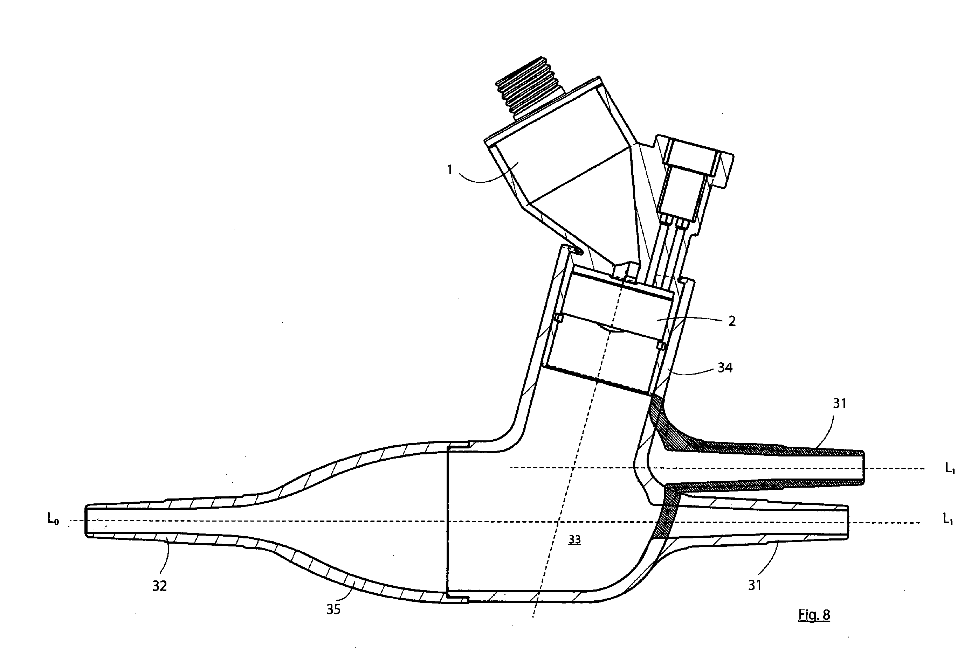

Referring to Fig. 8 an alternative arrangement is illustrated. In this case the axis of the inlet is above the axis at the outlet.

This connector gives higher drug deposition results as a result of the gentle taper and increased internal volume. Rain-out is reduced due to the taper and increased internal volume.

This device is used in conjunction with an insufflator to deliver aerosolised medication to the pneumoperitoneum along narrow bore tubing. The device is designed to address the widespread complications associated with minimally invasive surgery, and will create a greater distribution of medication on the organs to alleviate postoperative pain, adhesion formation and infection.

Sterile water may be used. In the case of an liquid solution any suitable solution may be used. Solutions with a salt concentration in the range 1µM (micro molar) to 154mM (milli molar) (0.9% saline) are optimum as they cover the majority of medical applications. In addition, such saline concentrations can be readily nebulised using the aerosolisation technology used in the invention.

Liquid, saline or water for humidifying purposes only and/or medicament, can be delivered into the nebulizer reservoir through the opening in the top of the nebulizer that is appropriately sized to receive standard nebules or alternatively may be applied by syringe or other delivery means. In another embodiment it would be possible to supply the nebulizer pre-loaded with medicament avoiding the requirement to separately add medicament to the system.

In addition to acting as a humidifying agent the nebulizer can also act to deliver any agent presented in an liquid drug solution. The system facilitates delivery of, for example, pain-relief medications, anti-infectives, anti-inflammatory and/or chemotherapy agents in aerosol form to the body cavity. These therapeutic agents could also act as humidifying substances in their own right.

The nebulised liquid entrained in the insufflation gas may contain any desired therapeutic and/or prophylactic agent. Such an agent may for example be one or more of an analgesic, an anti-inflammatory, an anaesthetic, an anti-infective such as an antibiotic, an anti-cancer chemotherapy agent, and/or any agent which interferes with processes that result in the adhesion function.

Typical local anaesthetics are, for example, Ropivacaine, Bupivacaine and Lidocaine.

Typical anti-infectives include antibiotics such as an aminoglycoside, a tetracycline, a fluroquinolone; anti-microbials such as a cephalosporin; and antifungals.

Anti-inflammatories may be of the steroidal or non-steroidal type.

Anti-cancer chemotherapy agents may be alkylating agents, antimetabolites anthracyclines, plant alkaloids, topoisomerase inhibitors, nitrosoureas, mitotic inhibitors, monoclonal antibodies, tyrosine kinase inhibitors, hormone therapies including corticosteroids, cancer vaccines, anti-estrogens, aromatase inhibitors, anti-androgens, anti-angiogenic agents and other antitumour agents.

The agent which interferes with the adhesion function may be any of those outlined in WO2005/092264A, the entire contents of which are herein incorporated by reference. In particular, the agent may be a crystalloid, hyaluronic acid, surfactant, phospholipid, polyethyleneglycol, Tranilast (N-(3¹,4¹-dimethoxycinnamoyl) anthranilic acid) or a Neurokinin 1 receptor (NK-1R) agonist, such as Aprepitant.

Typical analgesics include aspirin, acetaminophen, ibuprofen, naproxen, a Cox-2 inhibitor such as celecoxib, morphine, oxycodone and hydrocodone.

The system of the invention can be used for precise controlled delivery of drug and/or humidity during insufflation. No heating is required. Consequently there is no risk of damage to drugs due to heating. The system may be used to provide precise control over aerosol output can be exercised by utilising pulse rate control. The system may be used for targeted delivery of a range of drugs, thereby reducing systemic side effects. In addition the system provides alleviation of post-surgical pain experienced by the patient.

The nebuliser (or aerosol generator), has a vibratable member which is vibrated at ultrasonic frequencies to produce liquid droplets. Some specific, non-limiting examples of technologies for producing fine liquid droplets is by supplying liquid to an aperture plate having a plurality of tapered apertures extending between a first surface and a second surface thereof and vibrating the aperture plate to eject liquid droplets through the apertures. Such technologies are described generally in U.S. Pat. Nos. 5,164,740; 5,938,117; 5,586,550; 5,758,637; 6,014,970, 6,085,740, and US2005/021766A, the complete disclosures of which are incorporated herein by reference. However, it should be appreciated that the present invention is not limited for use only with such devices.

Various methods of controlling the operation of such nebulisers or aerosol generators are described in US6,540,154, US6,845,770, US5,938,117 and US6,546,927, the complete disclosures of which are incorporated herein by reference.

In use, the liquid to be aerosolised is received at the first surface, and the aerosol generator 2 generates the aerosolised first fluid at the second surface by ejecting droplets of the first fluid upon vibration of the vibratable member. The apertures in the vibratable member are sized to aerosolise the liquid by ejecting droplets of the liquid such that the majority of the droplets by mass have a size of less than 5 micrometers. The vibratable member 40 could be non-planar, and may be dome-shaped in geometry.

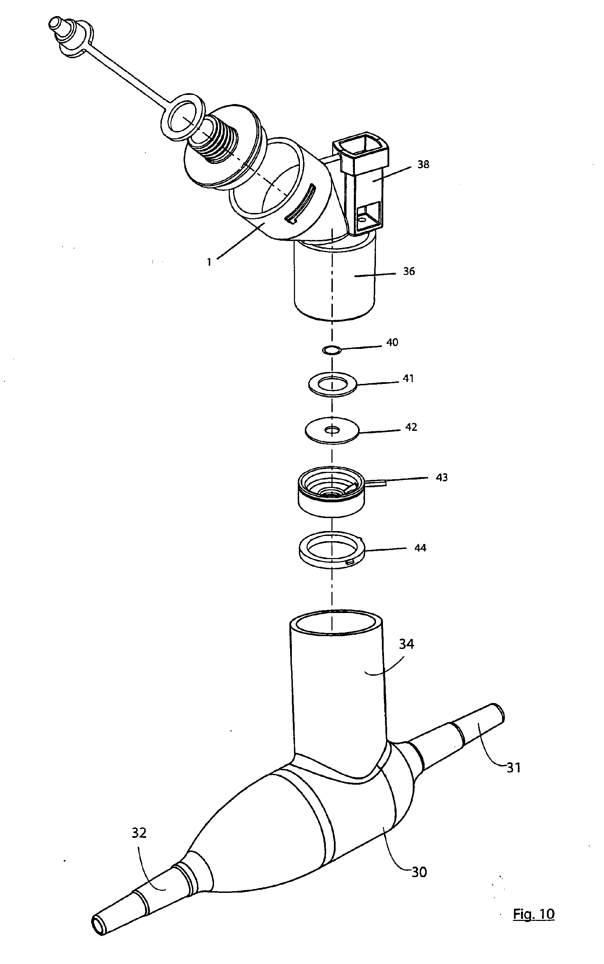

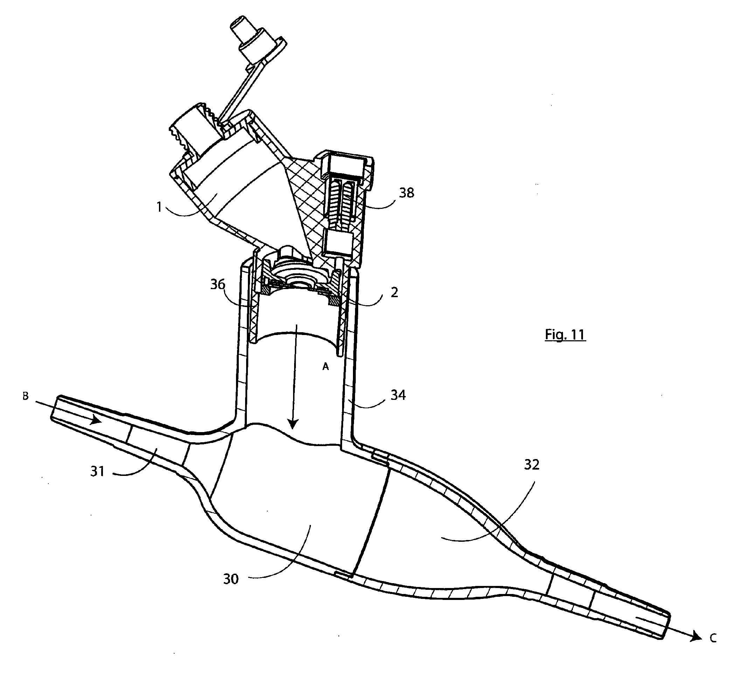

Referring particularly to Figs 10 and 11, in one case the aerosol generator 2 comprises a vibratable member 40, a piezoelectric element 41 and a washer 42, which are sealed within a silicone overmould 43 or by using O rings and secured in place within the housing 36 using a retaining ring 44. The vibratable member 40 has a plurality of tapered apertures extending between a first surface and a second surface thereof.

The first surface of the vibratable member 40, which in use faces upwardly, receives the liquid medicament from the reservoir 1 and the aerosolised medicament, is generated at the second surface of the vibratable member 40 by ejecting droplets of medicament upon vibration of the member 40. In use the second surface faces downwardly. In one case, the apertures in the vibratable member 40 may be sized to produce an aerosol in which the majority of the droplets by weight have a size of less than 5 micrometers.

The complete nebuliser may be supplied in sterile form, which is a significant advantage for a surgical device.

Referring to Fig. 1A, in this case liquid solution is fed from a reservoir 9 to the aerosol generator 2 along a delivery tube 13. In this case a flow rate sensor/meter 11 is located in the flow path of the insufflation gas from an insufflator 12 to the aerosol generator 2. The flow rate sensor/meter 11 is connected by a control wire 70 to the controller 3, and the aerosol generator 2 is connected to the controller 3 by a control wire 16. The flow rate sensor/meter 11 may be a hot wire anemometer, or in the case where the flow is laminar or can be laminarised, a differential pressure transducer.

Liquid solution may be stored in the reservoir I container of the nebuliser or the liquid solution may be delivered to the reservoir 1 of the aerosol generator 2 in this case from the supply reservoir 9 along the delivery line 13. The flow of liquid solution may be by gravity and/or may be assisted by an in-line flow controlling device 17 such as a pump and/or a valve which may be positioned in the delivery line 13. The operation of the flow controlling device 17 may be controlled by the controller 3 along a control wire 18 to ensure that the aerosol generator 2 has a supply of liquid solution during operation. The device 17 may be of any suitable type.

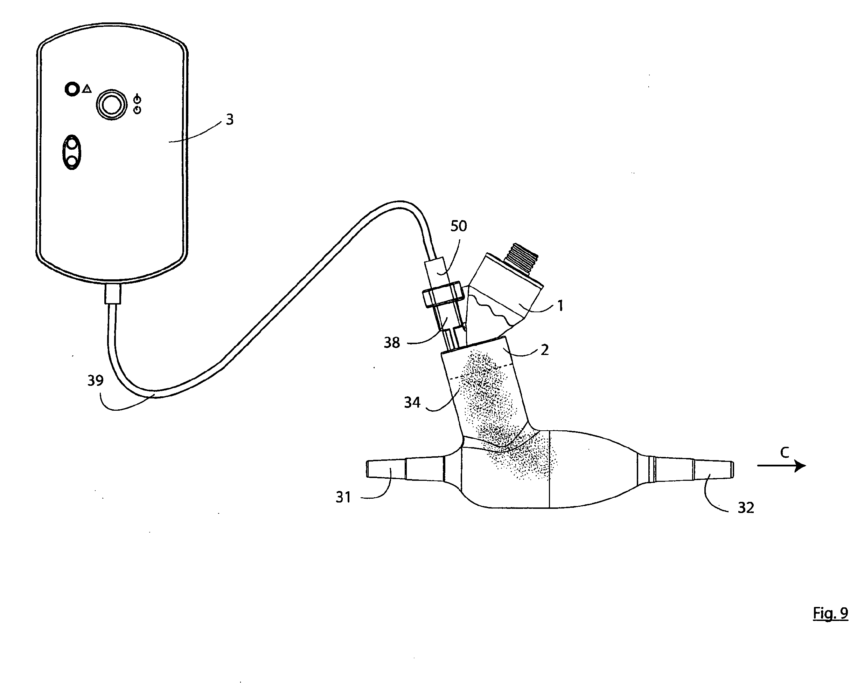

Referring particularly to Fig 9, the controller 3 controls operation of and provides a power supply to the aerosol generator 2. The aerosol generator has a housing which defines the reservoir 1. The housing has a signal interface port 38 fixed to the lower portion of the reservoir 1 to receive a control signal from the controller 3. The controller 3 may be connected to the signal interface port 38 by means of a control lead 39 which has a docking member 50 for mating with the port 38. A control signal and power may be passed from the controller 3 through the lead 39 and the port 38 to the aerosol generator 2 to control the operation of the aerosol generator 2 and to supply power to the aerosol generator 2 respectively.

The power source for the controller 3 may be an on-board power source, such as a rechargeable battery, or a remote power source, such as a mains power source, or an insufflator power source. When the remote power source is an AC mains power source, an AC-DC converter may be connected between the AC power source and the controller 3. A power connection lead may be provided to connect a power socket of the controller 3 with the remote power source.

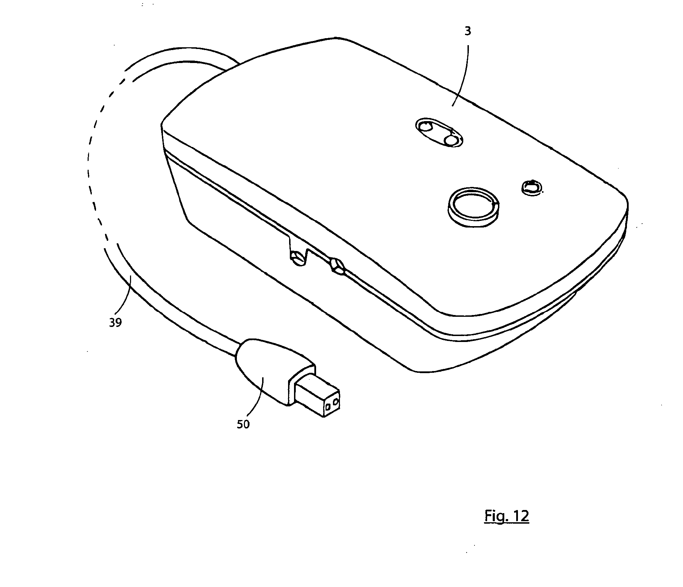

Referring particularly to Fig. 12 the controller 3 has a housing and a user interface to selectively control operation of the aerosol generator 2. Preferably the user interface is provided on the housing which, in use, is located remote from the aerosol generator housing. The user interface may be in the form of, for example, an on-off button. In one embodiment a button can be used to select pre-set values for simplicity of use. In another embodiment a dial mechanism can be used to select from a range of values from 0-100%.

Status indication means are also provided on the housing to indicate the operational state of the aerosol generator 2. For example, the status indication means may be in the form of two visible LED's, with one LED being used to indicate power and the other LED being used to indicate aerosol delivery. Alternatively one LED may be used to indicate an operational state of the aerosol generator 2, and the other LED may be used to indicate a rest state of the aerosol generator. 2.

A fault indicator may also be provided in the form of an LED on the housing. A battery charge indicator in the form of an LED may be provided at the side of the housing.

Referring particularly to Fig 1, the liquid solution in the reservoir flows by gravitational action towards the aerosol generator 2 at the lower medicament outlet. The controller 3 may then be activated to supply power and a control signal to the aerosol generator 2, which causes the piezoelectric element 41 to vibrate the non-planar member 40. This vibration of the non-planar member 40, causes the liquid solution at the top surface of the member 40 to pass through the apertures to the lower surface where the liquid solution is aerosolised by the ejection of small droplets of solution.

Referring particularly to Figs 10 and 11, the aerosol passes from the aerosol generator 2 into the neck 36 of the aerosol generator housing, which is mounted within the aerosol supply conduit of the connector 30 and into the gas conduit of the connector 30 (flow A). The aerosol is entrained in the insufflation gas conduit with gas, which passes into the gas conduit through the inlet 31 (flow B). The entrained mixture of the aerosol and the insufflation gas then passes out of the gas conduit through the outlet 32 (flow C) and on via an insufflator line 15 to a patient, for example into the abdomen of the patient.

In use during laparoscopic surgery the flow of the insufflation gas into the abdomen of a patient is commenced to insufflate the abdomen. The flow rate sensor/meter 11 determines the flow rate of the insufflation gas. In response to the fluid flow rate of the insufflation gas, the controller 3 commences operation of the aerosol generator 2 to aerosolise the liquid solution. The aerosolised liquid solution is entrained with the insufflation gas, and delivered into the abdomen of the patient to insufflate at least part of the abdomen.

In the event of alteration of the fluid flow rate of the insufflation gas, the flow rate sensor/meter 11 determines the alteration, and the controller 3 alters the pulse rate of the vibratable member of the nebuliser accordingly.

The controller 3 is in communication with the flow rate sensor/meter 11. The controller 3 is configured to control operation of the aerosol generator 2, responsive to the fluid flow rate of the insufflation gas and also independent of the fluid flow rate of the insufflation gas as required.

In one case, the controller 3 is configured to control operation of the aerosol generator 2 by controlling the pulse rate at a set frequency of vibration of the vibratable member, and thus controlling the fluid flow rate of the liquid solutions.

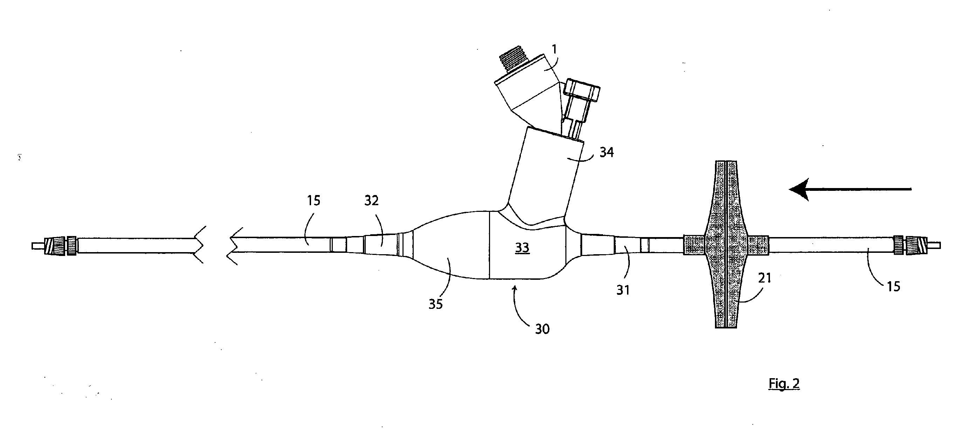

The controller 3 may comprise a microprocessor 4, a boost circuit 7, and a drive circuit 8. Fig. 2 illustrates the microprocessor 4, the boost circuit 7, the drive circuit 8 comprising impedance matching components (inductor), the nebuliser 2, and the aerosol. The inductor impedance is matched to the impedance of the piezoelectric element of the aerosol generator 2. The microprocessor 4 generates a square waveform of 128KHz which is sent to the drive circuit 8. The boost circuit 7 generates a 12V DC voltage required by the drive circuit 6 from an input of either a 4.5V battery or a 9V AC/DC adapter. The circuit is matched to the impedance of the piezo ceramic element to ensure enhanced energy transfer. A drive frequency of 128 KHz is generated to drive the nebuliser at close to its resonant frequency so that enough amplitude is generated to break off droplets and produce the aerosol. If this frequency is chopped at a lower frequency such that aerosol is generated for a short time and then stopped for a short time this gives good control of the nebuliser's flow rate. This lower frequency is called the pulse rate.

The drive frequency may be started and stopped as required using the microprocessor 4. This allows for control of flow rate by driving the nebuliser 2 for any required pulse rate. The microprocessor 4 may control the on and off times to an accuracy of milliseconds.

The nebuliser drive circuit consists of the electronic components designed to generate output sine waveform of approximately 100V AC which is fed to nebuliser 2 causing aerosol to be generated. The nebuliser drive circuit 6 uses inputs from microprocessor 4 and boost circuit 7 to achieve its output. The circuit is matched to the impedance of the piezo ceramic element to ensure good energy transfer.

The aerosol generator 2 may be configured to operate in a variety of different modes, such as continuous, and/or phasic, and/or optimised.

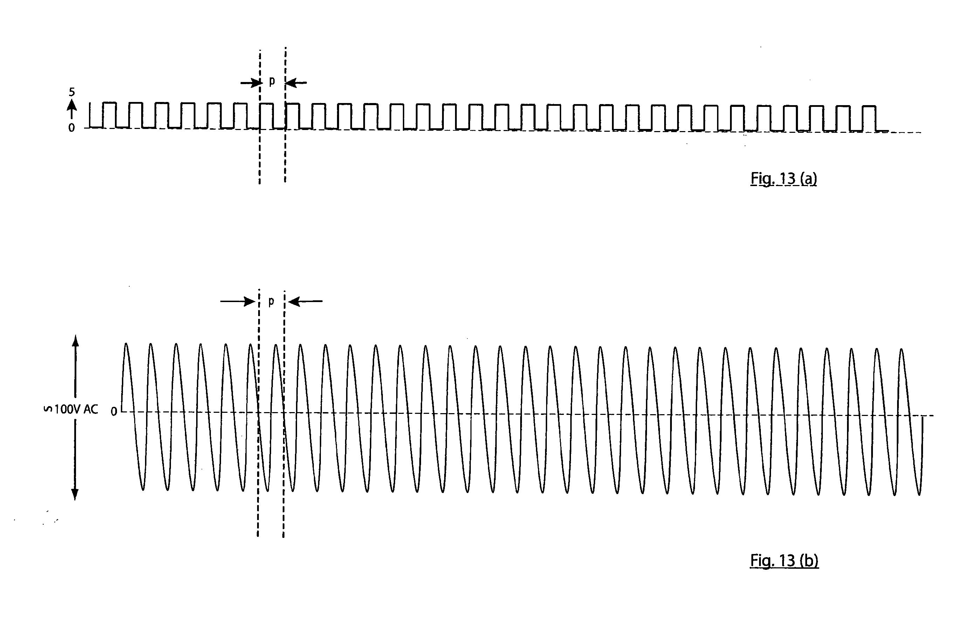

For example, referring to Fig 13(a) illustrates a 5V DC square waveform output from the microprocessor 4 to the drive circuit 6. Fig 7(b) shows a low power, ∼100V AC sine waveform output from drive circuit 8 to nebuliser 2. Both waveforms have a period p of 7.8µS giving them a frequency of 1/7.8µs which is approximately 128KHz. Both waveforms are continuous without any pulsing. The aerosol generator may be operated in this mode to achieve 100% aerosol output.

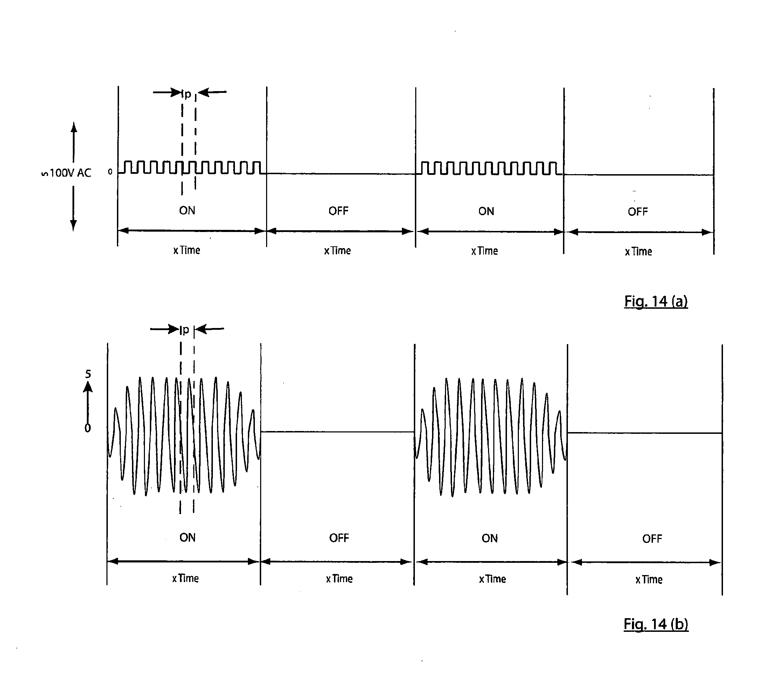

Referring to Figs 14(a) in another example, there is illustrated a 5V DC square waveform output from the microprocessor 4 to the drive circuit 8. Fig 14(b) shows a low power, ∼100V AC sine waveform output from the drive circuit 6 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time x is equal to the on time x. The aerosol generator may be operated in this mode to achieve 50% aerosol output.

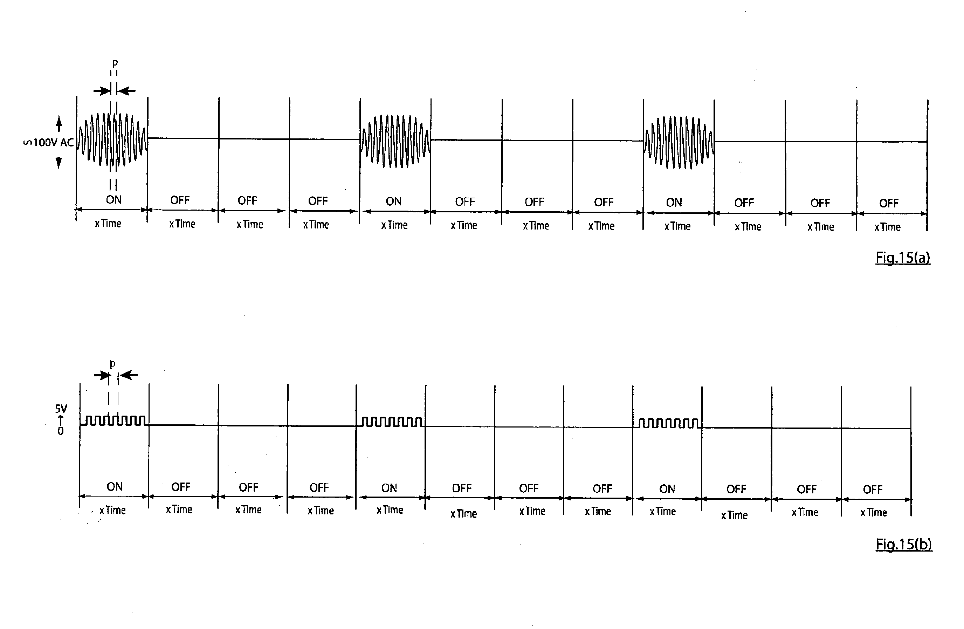

In another case, referring to Figs 15(a) there is illustrated a 5V DC square waveform output from microprocessor 4 to drive circuit 8. Fig 15(b) shows a low power, ∼100V AC sine waveform output from the drive circuit 8 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time is 3x while the on time is x. The aerosol generator may be operated in this mode to achieve 25% aerosol output.

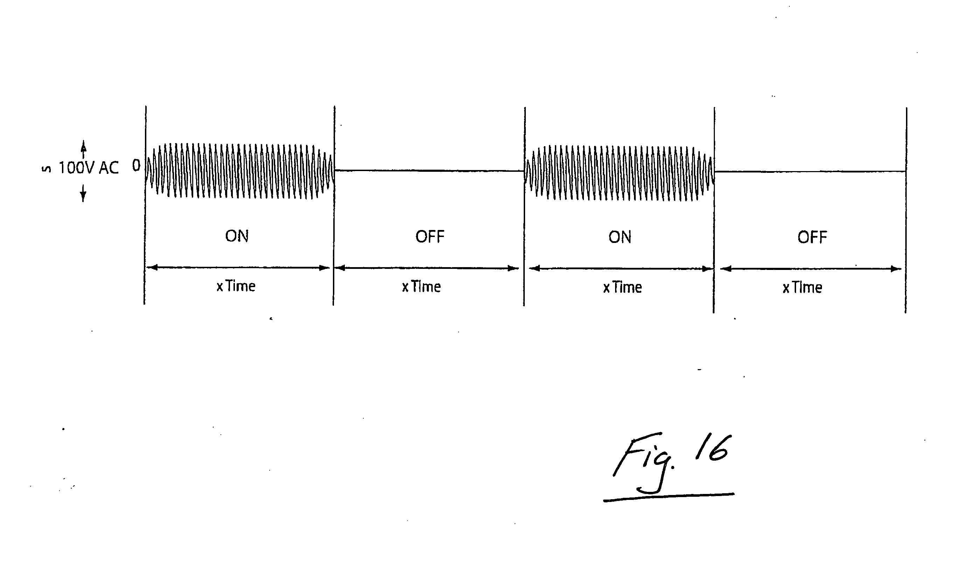

Referring to Fig 16, in one application pulsing is achieved by specifying an on-time and off-time for the vibration of the aperture plate. If the on-time is set to 200 vibrations and off-time is set to 200 vibrations, the pulse rate is 50% (½ on ½ off). This means that the flow rate is half of that of a fully driven aperture plate. Any number of vibrations can be specified but to achieve a linear relationship between flow rate and pulse rate a minimum number of on-time vibrations is specified since it takes a finite amount of time for the aperture plate to reach its maximum amplitude of vibrations.

The drive frequency can be started and stopped as required by the microprocessor; this allows control of flow rate by driving the nebuliser for any required pulse rate. The microprocessor can control the on and off times with an accuracy of microseconds.

A nebuliser can be calibrated at a certain pulse rate by measuring how long it takes to deliver a known quantity of solution. There is a linear relationship between the pulse rate and that nebuliser's flow rate. This allows accurate control of the rate of delivery of the aerosolised liquid solution. The ability to calibrate each nebulizer ensures that any inherent variation in output rate between each nebulizer can be eliminated. The output from each nebulizer when in-line in the insufflator circuit will be equivalent to a second nebulizer although the inherent flow rates of the two nebulizers are different. For example, to achieve a standard output of 0.044m1/min at 1Lmin from two nebulizers, one with an inherent output of 0.088ml/min and a second with an inherent output of 0.176m1/min the first nebulizer is controlled with a 50:50 on:off pulse rate, with the second set to a 25:75 on-off pulse rate so that both nebulizers give a 0.044ml/min output. This feature ensures that the nebulizers when located in the insufflation circuit have the potential to provide exactly the same rate of aerosol output as each other. This is possible because the amount of humidity a gas can hold is a known constant dependent on controllable factors.

The pulse rate may be lowered so that the velocity of the emerging aerosol is much reduced so that impaction rain-out is reduced.

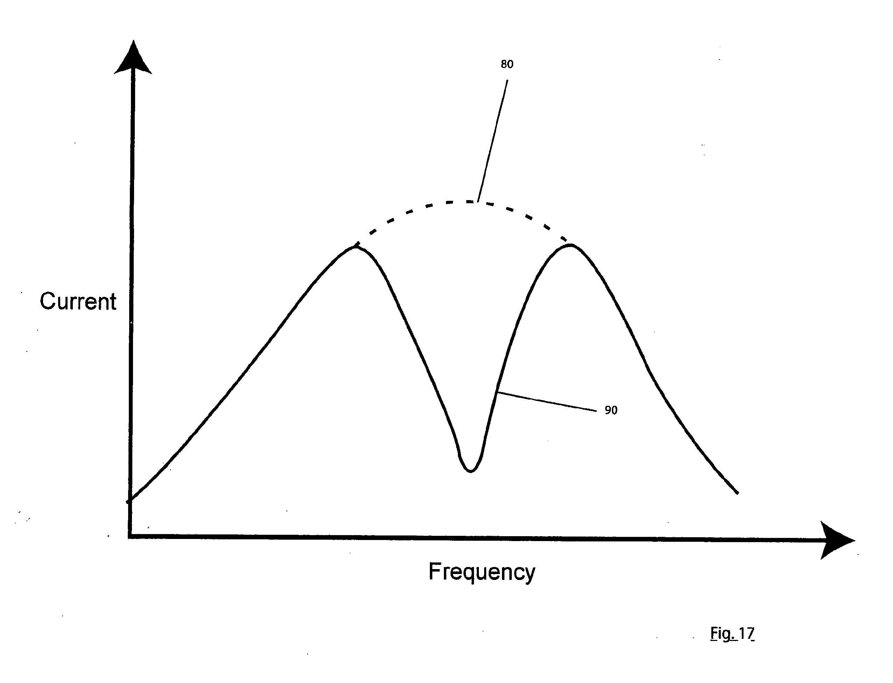

Detection of when the aperture plate is dry can be achieved by using the fact that a dry aperture plate has a well defined resonant frequency. If the drive frequency is swept from 120kHz to 145kHz and the current is measured then if a minimum current is detected less than a set value, the aperture plate must have gone dry. A wet aperture plate has no resonant frequency. The apparatus of the invention may be configured to determine whether there is any of the first fluid in contact with the aerosol generator 2. By determining an electrical characteristic of the aerosol generator 2, for example the current flowing through the aerosol generator 2, over a range of vibration frequencies, and comparing this electrical characteristic against a pre-defined set of data, it is possible to determine whether the aerosol generator 2 has any solution in contact with the aerosol generator 2. Fig. 17 illustrates a curve 80 of frequency versus current when there is some of the solution in contact with the aerosol generator 2, and illustrates a curve 90 of frequency versus current when there is none of the solution in contact with the aerosol generator 2. Fig. 17 illustrates the wet aperture plate curve 80 and the dry aperture plate curve 90.

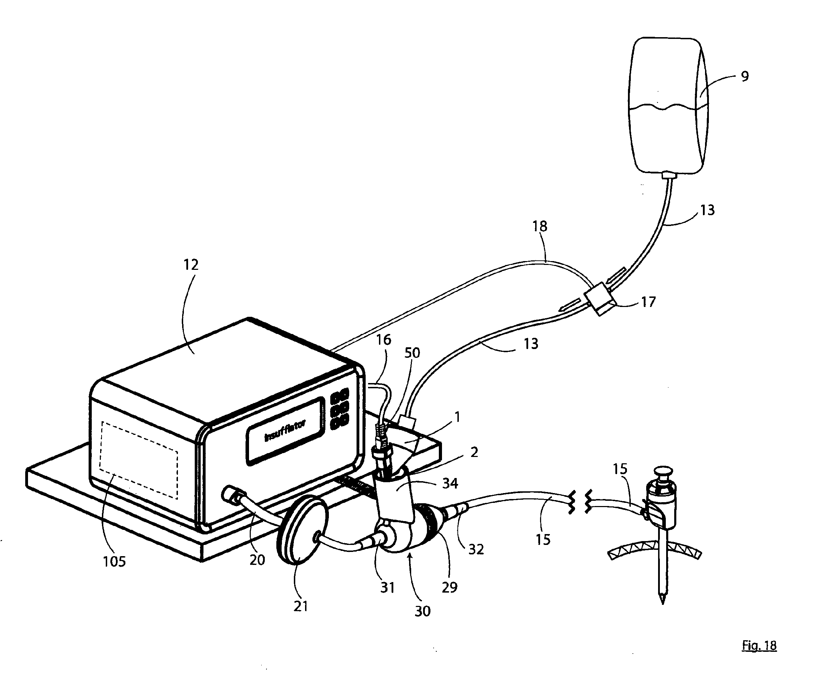

If an application requires a constant feed from a drip bag then a pump can be added in line to give fine control of the liquid delivery rate which can be nebulised drip by drip. The rate would be set so that liquid would not build up in the nebuliser. This system is particularly suitable for constant low dose delivery. Referring now to Fig. 18 there is illustrated another insufflation apparatus which is similar to the apparatus of Fig. 1 and like parts are arranged the same reference numerals. In this case the controller 3 is integrated into the insufflator 12. The insufflator 12 would have information on the rate of flow that it is producing and using an integrated circuit board may directly communicate with the nebuliser 2. This would eliminate the need for the separate flowmeter 11 and the stand-alone controller 3 to be present.

In another case there may be a common information bus between the insufflator 12 and the controller 3. The insufflator 12 would have information on the rate of flow that it is producing and would communicate this to the controller 3 and on to the nebuliser 2, thereby eliminating the need for the flowmeter 11. This would allow the invention to be backward compatible with a variety of types of insufflator.

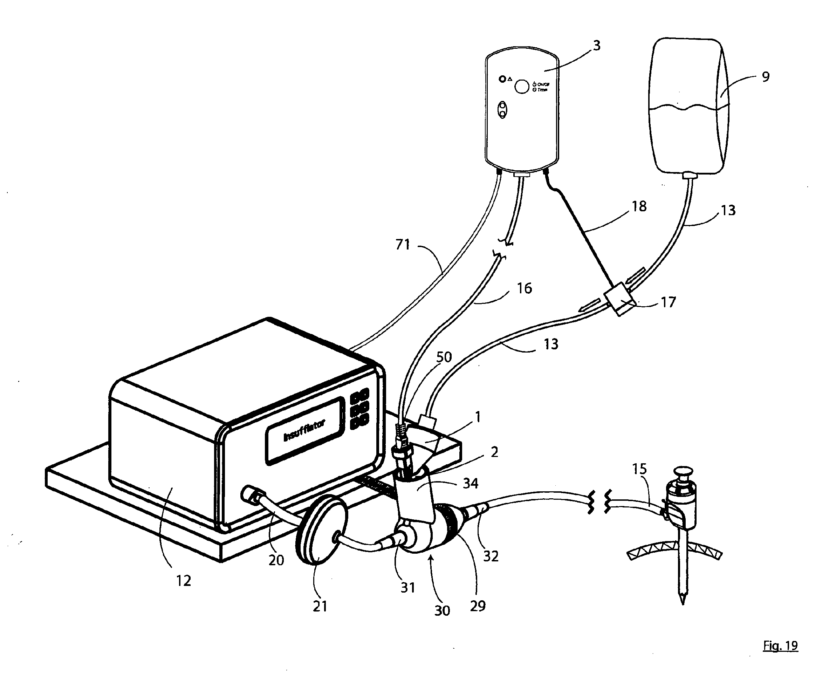

Referring to Fig. 19 there is illustrated another insufflation apparatus which is similar to the apparatus of Fig. 1 and like parts are again identified by the same reference numerals. In this case the insufflation gas flow signal is provided directly from the insufflator along a lead 71. One advantage of this arrangement is that no separate meter/sensor required.

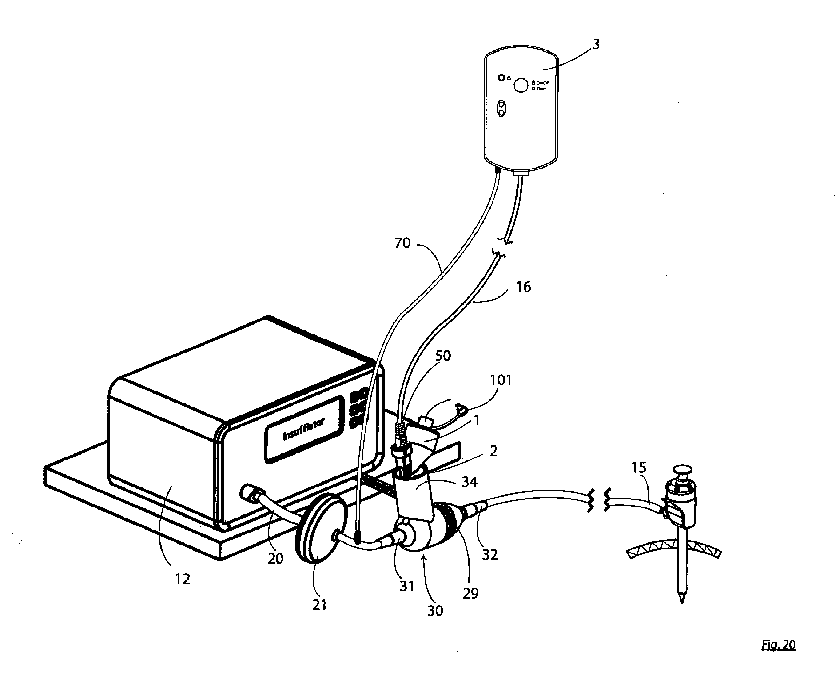

Referring to Fig. 20 there is illustrated another apparatus according to the invention which is similar to that illustrated in Fig. 1 and like parts are assigned the same reference numerals. In this case the nebuliser reservoir 1 has a top opening 100 which is closable by removable plug 101. Liquid, saline or water for humidifying purposes and/or medicament is delivered into the nebuliser reservoir through the opening 100. The opening 100 is appropriately sized to receive standard nebules containing liquid to be nebulised. The liquid may be applied by syringe or other suitable delivery means.

It is also possible to provide the nebuliser 1 pre-loaded with medicament to avoid the requirement to separately add medicament to the system.

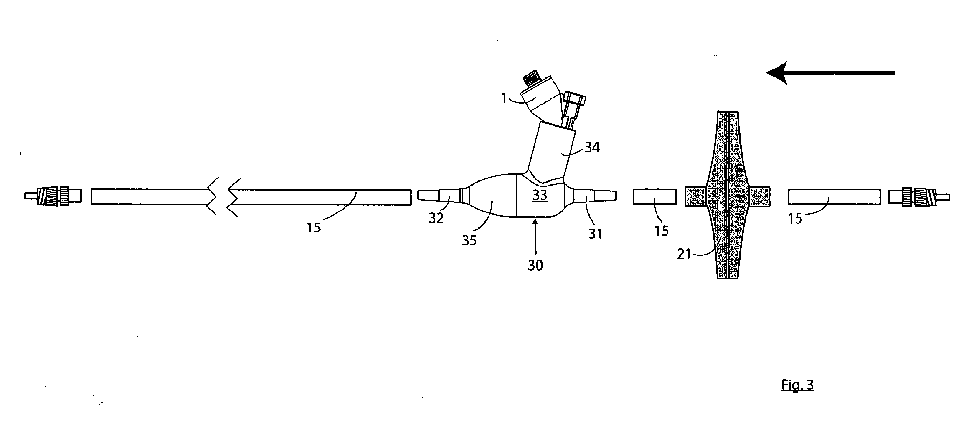

The apparatus of Fig. 20 is operated in a similar way to the modes of operation described above with reference to Figs. 2 to 11.

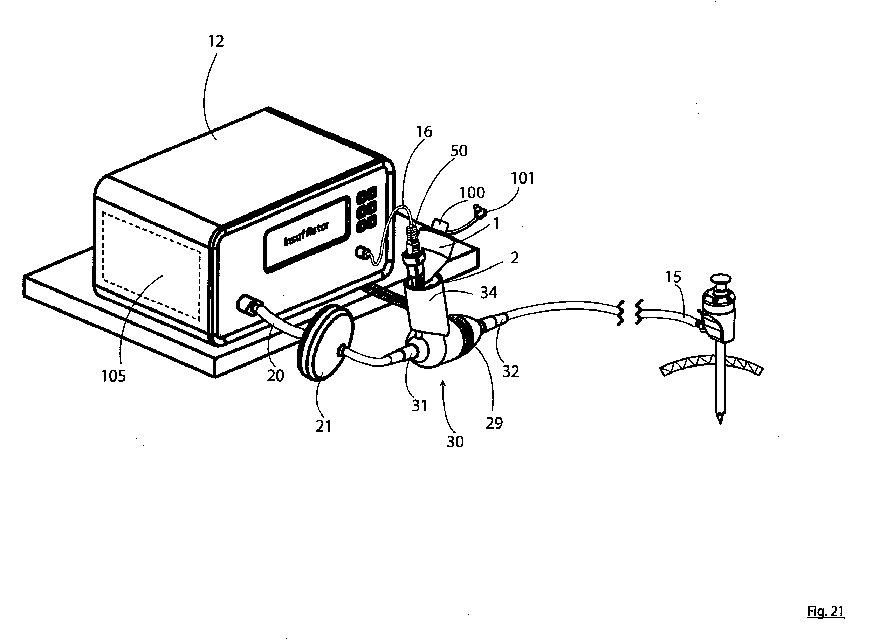

Referring to Fig. 21 there is illustrated another apparatus of the invention which is similar to that described above with reference to Fig. 12 and like parts are assigned the same reference numerals. In this case the nebuliser reservoir 1 has a top opening 100 and a removable plug/lid 101 as described with reference to Fig. 14 and the apparatus is operated as described above with the liquid being introduced through the opening 100. Again the nebuliser may be pre-loaded with medicament.

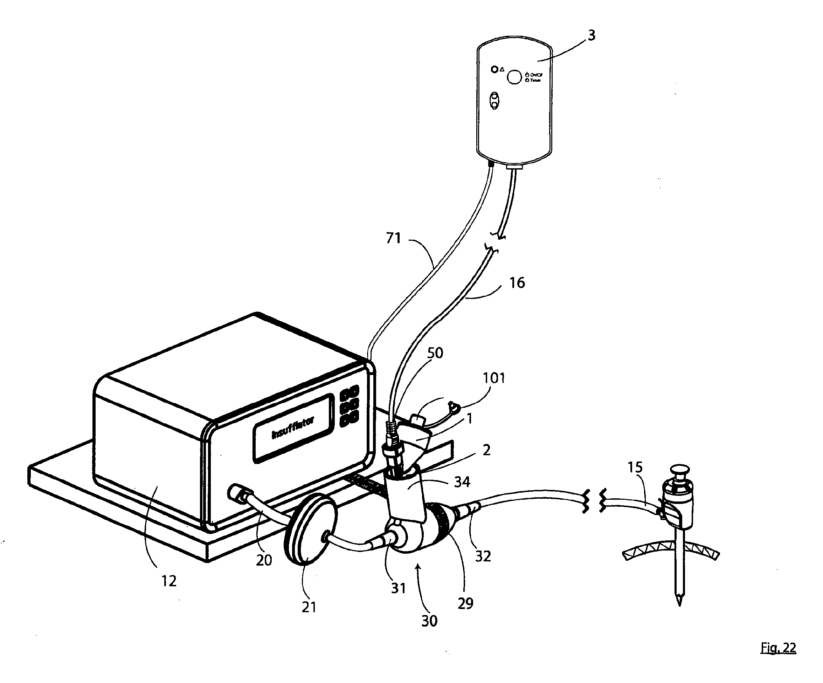

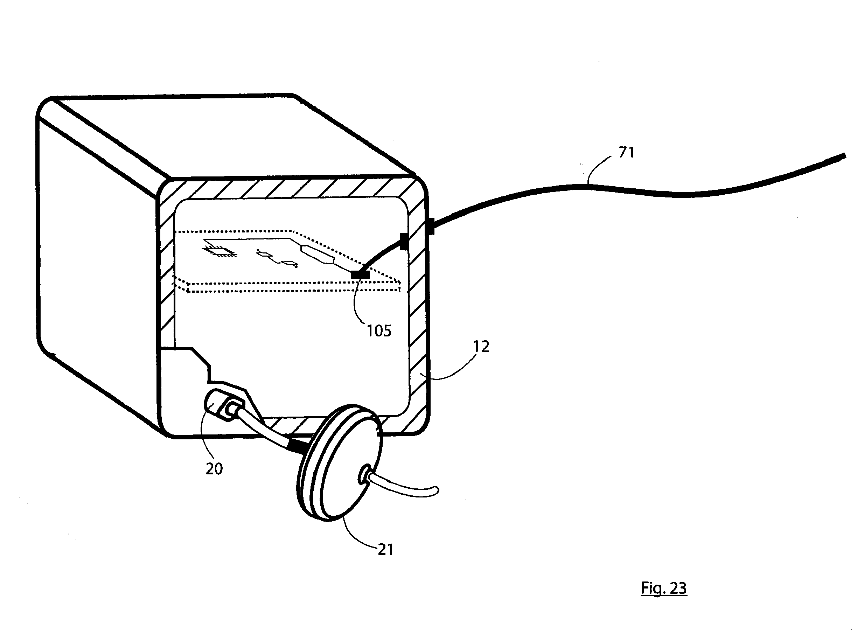

Referring to Fig. 22 there is illustrated another apparatus of the invention which is similar to that described above with reference to Fig. 13 and like parts are assigned the same reference numerals. In this case the nebuliser reservoir 1 again has a top opening 100 and a removable lid 101 as described with reference to Fig. 20 and the apparatus is operated as described above with the liquid being introduced through the opening 100. The nebuliser may be pre-loaded with medicament. The apparatus is operated as described above. Fig. 23 shows the connection of the controller lead 71 to the control circuit 105 of the insufflator 12.

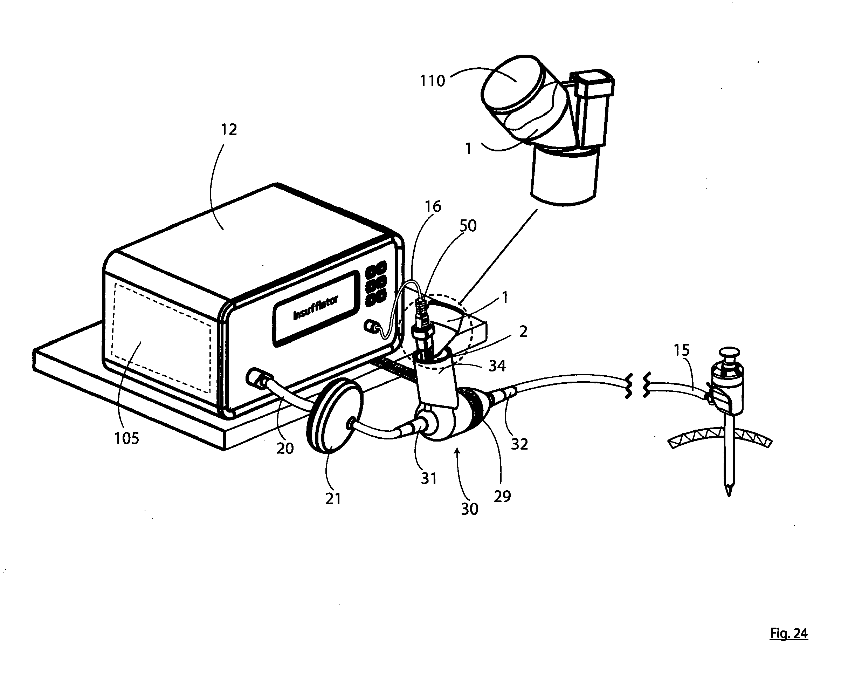

Referring to Fig. 24 there is illustrated a further apparatus according to the invention which is similar to those described above and like parts are assigned the same reference numerals. In this case the nebuliser reservoir 1 is closed by a lid 110 and the nebuliser is pre-loaded with medicament/liquid which avoids the requirement to separately add medicament to the system.

Humidity may be generated via the aerosolisation of any liquid solution. Relative humidity in the 50-100% range would be optimum. The control module can generate a nebuliser output of any defined relative humidity percentage based on the insufflator flow. These solutions include any liquid drug solution. Solutions with salt concentrations in the range 1µM― 154mM would be optimum.

The use of the nebulizer to humidify the insufflation gas prior to entering the body will eliminate the need for the body to humidify the gas once it is inside the body, thereby minimizing body heat loss by internal evaporation.

The control in nebulizer output allows proportional delivery of the required amount of humidity according to the amount of insufflation gas entering the body. In addition this control of aerosolization rate will prevent overloading of the insufflation gas with aerosol which would obscure the surgeons view.

The invention provides a system that can deliver different flow rates at different stages of the surgical procedure. Examples of such different flow rates include:
(i) delivering at 100% at the start of the procedure (Bolus);
(ii) delivering at a much lower rate say 5% during the procedure itself (Lower flow rate avoid fogging);
(iii) delivering at 100% at the end of the procedure (Bolus);
(iv) any combination of the above sequencing with variable % values.

In one case the controller which controls the operation of the aerosol generator is pre-set to deliver a set amount of aerosol into the insufflation gas. For example, the controller may be set to deliver an amount of 5% into a flow of 1 litre per minute of insufflation gas to avoid fogging. The controller may be pre-set in the factory to operate in this manner. Alternatively there may be a user interface such as a switch, or keypad which may be used to change the setting. In these arrangements control responsive to an insufflation gas flow sensor is not essential.

There may be a clinical benefit in delivering a combination of anti-adhesion drugs/ anaesthetics/other therapeutics. Depending on the surgery type, selected drive profile and/or surgeons/anaesthesiologists preference the nebuliser may not have finished delivering the first therapeutic chosen by the time the second therapeutic is required. Depending on the therapeutics chosen there may be issues resulting from mixing the two within the nebuliser reservoir while in liquid form creating difficulties in delivering both simultaneously or in series. This problem may be overcome by incorporating a second nebuliser into the circuit.

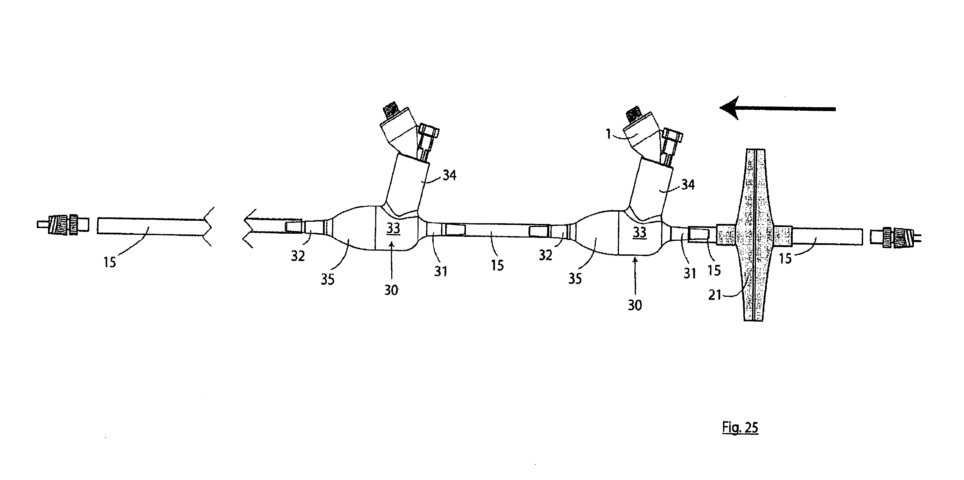

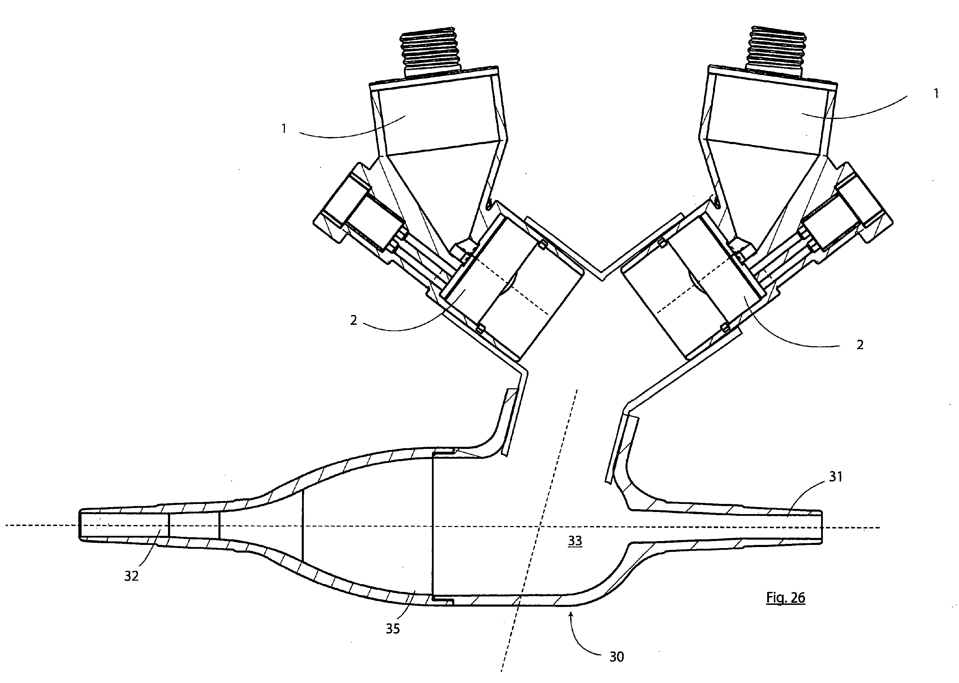

Two methods of incorporating this dual nebuliser functionality are as follows:
1. Two of the T-pieces connections are placed one after the other in line. This is illustrated in Fig. 25.
2. One T-piece connection is used but at the mounting leg there is a Y-Piece with two limbs; effectively turning the single mounting leg into two mounting legs. This is illustrated in Fig. 26.

It may be desirable to have delivery of one therapeutic (at 100% output) during the pulsed (5% delivery) delivery of a humidification agent, incorporating a second nebuliser facilitates this.

The system need not be located in the direct flow path of insufflation gas. In addition, minimal caregiver intervention during laparoscopic procedure is required. The system is small and compact and allows for integration with an insufflator.

The device of the invention can be used throughout the procedure carried out by a surgeon. The device ensures that humidity is actively controlled during the procedure and thus ensures that a surgeon's view is clear as fogging is avoided.

In the system of the invention the nebuliser output is controlled by pulsing to provide delivery of humidity and/or medicament into the insufflation gas during surgery without causing fogging.

The control may be provided either by providing a maximum output limit on the nebuliser or by linking directly to the insufflator flow.

All parts of the device (except the controller and associated leads) are autoclavable which provides a significant advantage for a device used in surgery.

The invention is not limited to the embodiments hereinbefore described which may be varied in construction and detail.

## Claims

1. A connector for an insufflation system comprising a housing having a mixing chamber, an insufflator gas inlet to the mixing chamber, an aerosolised liquid inlet to the mixing chamber, an outlet for delivery of insufflation gas with aerosolised liquid entrained therein, the connector causing a substantial reduction in the velocity of insufflation gas from the insufflation gas inlet as it enters the mixing chamber and causing an increase in the velocity of the insufflation gas with entrained aerosol between the mixing chamber and the outlet.

2. A connector as claimed in claim 1 comprising a tapered portion leading from the mixing chamber to the outlet.

3. A connector as claimed in claim 2 wherein the taper angle β of the tapered portion is between about 10° and 30°.

4. A connector as claimed in claim 2 or 3 wherein the tapered portion has an entry with a first internal diameter (D₁) adjacent to the mixing chamber and a second internal diameter (D₂) at the outlet and the ratio of the first diameter to the second diameter is at least 2:1.

5. A connector as claimed in claim 4 wherein said ratio D₁:D₂ is at least 3:1; at least 4:1; at least 5:1.

6. A connector as claimed in claim 4 or 5 wherein the distance between the taper entry and the outlet defines a length (L) and the ratio of the length to the first diameter D₁ is at least 2:1.

7. A connector as claimed in claim 6 wherein said ratio L:D₁ is at least 3:1; at least 4:1.

8. A connector as claimed in any of claims 1 to 7 wherein a central longitudinal axis L₁ at the insufflation gas inlet is coaxial with a central longitudinal axis L₀ at the outlet.

9. A connector as claimed in any of claims 1 to 7 wherein a central longitudinal axis L₁ at the insufflation gas inlet is above a central longitudinal axis L₀ at the outlet.

10. A connector as claimed in any of claims 1 to 9 comprising a mounting leg for mounting an aerosol generator device to the connector.

11. A connector as claimed in claim 10 comprising an interlock between the connector mounting leg and the aerosol generator device.

12. A connector as claimed in claim 10 or 11 wherein the mounting leg is angled towards the inlet of the connector.

13. A connector as claimed in claim 12 wherein the mounting leg is offset from a vertical axis by a tilt angle of at least 10°, about 15°.

14. Apparatus from use in insufflation comprising a connector as claimed in any of claims 1 to 13 and an aerosol generator for aerosolising a liquid and entraining the aerosol within an insufflation gas, the aerosol generator comprising a vibratable member having a plurality of apertures extending between a first surface and a second surface, the first surface being adapted to receive the fluid to be aerosolised, the aerosol generator being configured to generate an aerosol at the second surface.

15. A method for carrying out a procedure involving insufflation comprising the steps of:
generating an insufflation gas;
reducing the velocity of the insufflation gas on entry to a mixing chamber;
aerosolising a fluid using an aerosol generator;
entraining the aerosol with the insufflation gas in the mixing chamber;
increasing the velocity of the insufflation gas with entrained aerosol; and
delivering the insufflation gas with entrained aerosol.
